# FASCICULE DE BREVET EUROPEEN

(11) **EP 1 537 377 B1**
(45) Date de publication et mention de la délivrance du brevet: **04.07.2018**
(21) Numéro de dépôt: 03769578.0
(22) Date de dépôt: 05.09.2003
(51) Int. Cl.: G01B 7/02, A61B 6/10, H03K 17/955

(54) **DÉTECTEUR DE PROXIMITÉ PAR CAPTEUR CAPACITIF**
NÄHERUNGSDETEKTOR MIT KAPAZITIVEM SENSOR
PROXIMITY DETECTOR COMPRISING CAPACITIVE SENSOR

(30) Priorité: 06.09.2002 FR 0211089
(43) Date de publication de la demande: 08.06.2005
(73) Titulaire: Fogale Nanotech, 30900 Nîmes (FR)
(72) Inventeur: ROZIERE, Didier, F-30900 Nîmes (FR)
(74) Mandataire: Pontet Allano & Associes
(86) Numéro de dépôt international: PCT/FR2003/002654
(87) Numéro de publication internationale: WO 2004/023067

(56) Documents cités:
- FR-A- 2 756 048
- GB-A- 2 312 514
- US-A- 4 419 713
- US-A- 5 315 884
- US-A- 5 373 245
- US-A- 5 430 381
- US-A- 5 651 044
- US-A- 5 883 935
- US-A- 5 952 835
- US-B1- 6 225 939
- US-B1- 6 348 862

## Description

La présente invention concerne un détecteur de proximité par capteur capacitif.

Dans de nombreuses applications industrielles il est nécessaire de détecter et mesurer la proximité entre une machine et un obstacle, que celui-ci soit un autre objet ou un individu, afin de délivrer une information de distance de proximité, des signaux d'alerte et agir en conséquence.

A titre d'exemple non limitatif d'application industrielle de ce type de détecteur l'on peut citer la gestion d'une fonction anti-collision entre des robots mobiles ou à poste fixe avec un obstacle, la gestion d'une fonction anti-effraction, et plus généralement toute gestion mettant en oeuvre une détection de proximité.

Dans le domaine médical de nombreux robots utilisés pour l'auscultation des patients nécessitent de connaître la position du patient par rapport aux éléments mobiles de la machine.

A titre d'exemple, pour des applications en radiologie ou en imagerie ou encore pour un traitement médical ou chirurgical il est essentiel de pouvoir fournir à l'opérateur d'un équipement ou de systèmes de contrôle automatisés des informations aussi précises que possible sur la position du patient afin de positionner rapidement et correctement les éléments d'auscultation.

Sur les systèmes de radiologie par rayons X, une connaissance en temps réel et millimétrique de la position d'un équipement de radiologie par rapport à un patient et à son environnement matériel immédiat permettrait d'accroître la vitesse des mouvements de la machine, d'améliorer la sécurité, et de minimiser les temps d'exposition aux rayons X.

Accroître la vitesse des mouvements des positionneurs vasculaires tout en garantissant une non collision avec le patient est un des souhaits actuels. Cependant, la physionomie du patient et de sa position par rapport au référentiel de la machine n'étant pas connue, les vitesses de déplacement de ces robots sont faibles afin que les pièces mobiles de la machine ne blessent pas accidentellement le patient. Généralement un arrêt d'urgence constitué par des interrupteurs mécaniques stoppe tous les mouvements quand le détecteur ou l'émetteur X arrivent au contact du patient ou d'une autre partie de l'équipement. Cependant la cinétique des objets en mouvements et la faible course des contacteurs imposent des vitesses de déplacements faibles. L'augmentation de la vitesse du robot n'est alors possible que si un dispositif sans contact détecte le patient à une distance, dite seuil haut, suffisante pour ralentir les mouvements avant d'arriver au contact du patient. Une distance minimale, dite seuil bas, permet de réaliser la fonction d'arrêt d'urgence anti-collision.

Il existe donc actuellement un besoin réel pour des détecteurs de proximité sans contact fournissant une information de distance précise et utilisables dans des environnements spécifiques tels que celui de l'imagerie médicale. Les documents US 4,987,583, WO 9730633 et WO 9719638 divulguent des détecteurs de proximité adaptés à ce type d'application.

Le document US 5,952,835 divulgue un détecteur de proximité sans contact. L'électronique mise en oeuvre est un détecteur tout ou rien fonctionnant à l'aide d'un oscillateur à transistor FET connecté à une électrode de mesure non gardée.

Le document US 5,442,347 divulgue un détecteur de proximité de type capacitif à double garde commandée, fonctionnant en mesure différentielle de phase en exploitant des constantes RC générées avec des résistances de référence. Une garde est créée en reproduisant le signal capteur à l'aide d'une mémoire tampon (buffer). Or, un problème de principe majeur apparaît dans ce concept car le buffer rajoute une capacité parasite à la capacité à mesurer. Cette capacité parasite est bien supérieure à la capacité à mesurer ce qui engendre des erreurs de mesure et des instabilités importantes.

Le document US 5,554,973 divulgue lui un détecteur électrostatique de type capacitif opérant selon un principe de fonctionnement à capacités commutées, sans garde.

Le document US 6,348,862 divulgue un détecteur de proximité incluant une électrode de détection et une pluralité d'électrodes de commande situées à proximité d'une région spatiale dans laquelle est situé un objet à détecter.

Enfin, le document US 5 651 044 A décrit un détecteur suivant le préambule de la revendication 1.

Un objectif principal de la présente invention est de proposer un détecteur de proximité par capteur capacitif qui fournit une mesure précise (typiquement millimétrique) de la position d'un objet à une portée de mesure (typiquement décimétrique) supérieure à celle permise par les détecteurs de proximité de l'art antérieur, avec notamment pour effet de permettre d'augmenter la vitesse de déplacement des machines de radiologie et de fournir une topographie du patient ayant pour but d'évaluer son épaisseur afin d'optimiser la puissance d'émission des faisceaux X et ainsi de minimiser le niveau de radiation nécessaire pour réaliser une image.

Cet objectif est atteint avec un détecteur capacitif de proximité selon la revendication 1.

On peut ainsi réaliser l'équivalent d'une caméra à pixels dans laquelle chaque pixel est constitué par une électrode. Cette caméra déplacée le long du corps d'un patient va permettre de réaliser une topographie de ce patient afin d'obtenir une mesure de son épaisseur.

Le pont capacitif flottant peut avantageusement être du type divulgué dans le document FR2756048. On peut aussi utiliser une chaîne de mesure capacitive du type décrit dans le document FR2640373, qui met en oeuvre une source de tension de polarisation et un transformateur triaxial.

Le détecteur de proximité selon l'invention, constitué d'une pluralité d'électrodes de mesure orientées suivant plusieurs axes afin de couvrir toutes les zones utiles, peut être réalisé en plusieurs antennes de détection.

Dans une forme avantageuse de réalisation d'un détecteur de proximité selon l'invention, l'antenne de détection comprend en outre une garde unique pour l'ensemble des électrodes de mesure de l'antenne.

Mais on peut aussi prévoir une configuration dans laquelle l'antenne de détection comprend en outre plusieurs gardes prévues chacune pour une partie de l'ensemble des électrodes de mesure de l'antenne.

Les antennes de détection peuvent être réalisées à l'aide d'un circuit rigide ou souple et connectées aux moyens électroniques.

Les moyens électroniques et les moyens numériques de pilotage et de calcul peuvent coopérer pour mesurer une distance successivement sur chaque électrode d'une antenne, selon un ordre prédéterminé et modifiable.

Les antennes de détection comportent de préférence une piste de test placée à l'arrière ou à proximité des électrodes (côté plan de garde), qui, en fonctionnement normal, est au potentiel de la garde, et, en test, est mise à la masse.

Dans ces conditions, chaque électrode voit une capacité parasite simulant la présence d'un objet, afin de vérifier l'intégrité du détecteur de proximité.

Les moyens électroniques et les moyens numériques de pilotage et de calcul coopèrent pour délivrer un signal d'alarme indiquant une mesure incohérente ou un dysfonctionnement des moyens numériques de pilotage et de calcul.

On peut aussi prévoir que les moyens électroniques comprennent une ou plusieurs capacités de référence permettant de contrôler la calibration de l'électronique ou d'effectuer une recalibration automatique.

Dans une configuration particulière d'un détecteur de proximité selon l'invention, on peut placer, à proximité des électrodes de mesure, des surfaces de garde ou de masse disposées pour modifier les lignes de champ de ces électrodes. On peut ainsi créer des formes particulières de surface équivalente de ces électrodes de mesure.

Dans un mode particulier de réalisation, le détecteur de proximité selon l'invention est disposé sur la surface intérieure ou extérieure d'un capot ou boîtier habillant par exemple le détecteur d'imagerie X ou l'émetteur X.

Les moyens électroniques et les moyens numériques de pilotage et de calcul coopèrent pour délivrer des signaux d'alarme de seuil de détection de proximité. Les distances mesurées entre les électrodes et les objets détectés sont délivrées sous forme numérique et analogique.

Pour asservir les déplacements suivant les six degrés de liberté, des antennes sont par exemple disposées sur cinq faces du boîtier ou capot.

Lorsqu'il s'agit d'un détecteur de proximité mis en oeuvre dans un équipement de radiologue par rayons X comprenant un dispositif pour émettre un faisceau X prévu pour irradier un objet ou un corps, une antenne, dite antenne X, est alors au moins partiellement traversée par le faisceau X.

Dans une configuration simple, l'antenne X peut par exemple comporter un perçage permettant le passage du faisceau X. Cette zone est alors non mesurante car non pourvue d'électrodes.

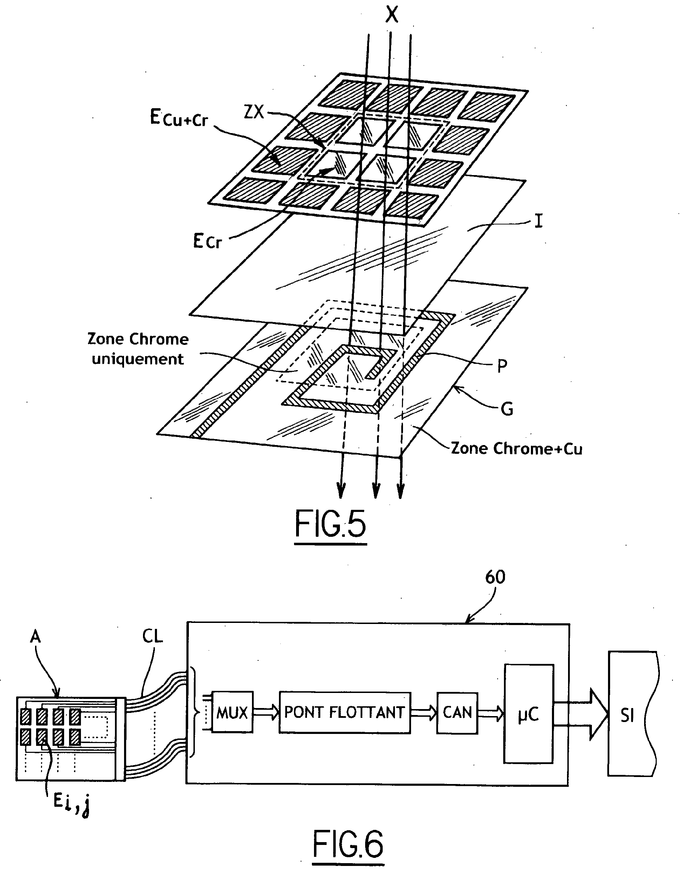

Pour pallier cet inconvénient on peut alors prévoir que l'antenne X soit, dans la zone du faisceau X, réalisée avec des matériaux au moins partiellement transparent au rayon X. Cette réalisation est possible à partir d'un circuit imprimé souple composé d'un isolant métallisé sur ses deux faces par une couche très mince de chrome constituant la couche d'accrochage d'une couche de cuivre, cette couche de cuivre est supprimée par attaque chimique sur la zone qui correspond au passage du faisceau X afin de ne laisser sur l'isolant que la couche mince de chrome dans laquelle des pistes de liaison, des électrodes capacitives, la piste de test et la garde sont réalisées (Fig. 5). L'émetteur X peut aussi être pourvu d'antennes dites X.

Cette dernière configuration permet de recouvrir complètement le détecteur d'électrodes pour augmenter l'efficacité de ce détecteur.

Le détecteur de proximité selon l'invention se distingue notamment du détecteur divulgué dans le document US 5,952,835 par le fait que dans la présente invention, l'électronique fonctionne en mesure d'amplitude avec une garde, et que l'oscillateur possède des caractéristiques constantes indépendantes de la capacité à mesurer. De plus, le détecteur selon l'invention fonctionne en mesure d'amplitude et non en mesure différentielle de phase.

Ce dispositif détecteur de proximité permet d'augmenter la vitesse de déplacement des machines actuelles de radiologie, de détection de sécurité (anticollision), d'effectuer une image grossière en trois dimensions du patient, d'évaluer l'épaisseur d'un patient afin d'optimiser la puissance des rayon X pour réaliser des images avec un minimum de radiation, et d'améliorer la qualité d'image.

Le détecteur capacitif de proximité selon l'invention permet de piloter l'approche d'un positionneur vasculaire pour application médicale, à l'aide de plusieurs antennes, équipées d'une multitude d'électrodes capacitives, logées dans le détecteur. Ce dispositif effectue en temps réel la mesure de plusieurs distances absolues (une distance par électrode) séparant la surface du capot du détecteur et les objets environnants comme un patient ou la table.

Des dispositifs détecteurs de proximité selon l'invention peuvent aussi être mis en oeuvre dans des machines ou robots en mouvement, notamment des machines-outils, des robots industriels, des véhicules de transport, etc.... avec pour effet d'augmenter leur vitesse de fonctionnement et d'améliorer la sécurité.

D'autres avantages et caractéristiques de l'invention apparaîtront à l'examen de la description détaillée d'un mode de mise en oeuvre nullement limitatif, et des dessins annexés sur lesquels :
- la figure 1 est une vue synoptique schématique d'un équipement de radiographie intégrant deux détecteurs de proximité selon l'invention ;
- la figure 2 illustre l'agencement d'une antenne au sein d'un détecteur de proximité selon l'invention ;
- la figure 3 illustre un exemple de structure d'une antenne d'un détecteur de proximité selon l'invention ;
- la figure 4 représente schématiquement les entrées et sorties d'une carte électronique équipant un détecteur de proximité selon l'invention ; et
- la figure 5 illustre la structure d'un circuit souple utilisé pour la réalisation d'une antenne au sein d'un détecteur de proximité ; et
- la figure 6 illustre la structure d'une chaîne de mesure équipant un détecteur de proximité selon l'invention.

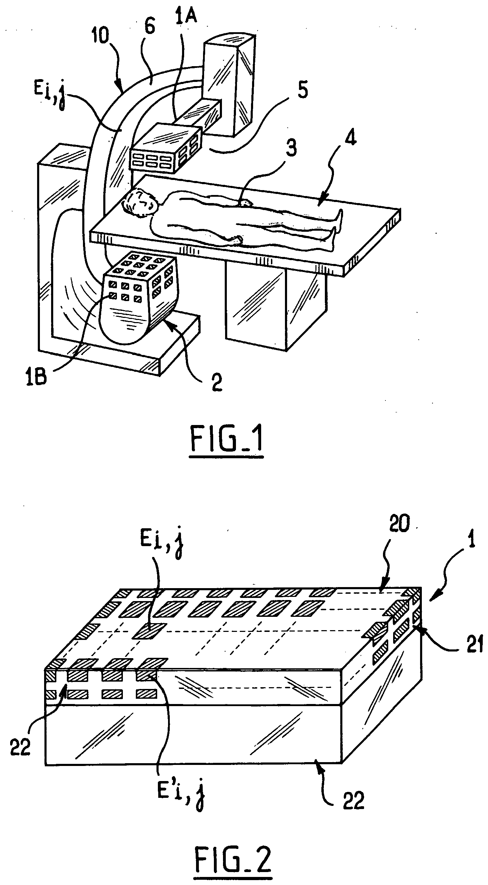

On va maintenant décrire, en référence à la figure 1, un exemple de mise en oeuvre de détecteurs de proximité selon l'invention dans une machine de radiologie X, pour un positionneur vasculaire.

Un premier détecteur de proximité (1A) est disposé à l'intérieur d'un dispositif détecteur X (5) équipant une machine de radiologie (10), et comporte plusieurs antennes tapissant cinq des parois intérieures ou extérieures du capot du détecteur X, chaque antenne comportant une pluralité d'électrodes Ei,j. Un second détecteur de proximité (1B) est disposé sur la surface intérieure du dispositif émetteur X (2) de la machine (10). Le dispositif émetteur X (2) et le dispositif détecteur X (5) sont installés aux deux extrémités d'une pièce mobile en forme de C mobile en rotation autour d'une table d'examen (4) sur laquelle un patient (3) est allongé.

En référence à la figure 2, un détecteur de proximité selon l'invention 1 comprend une antenne 20 disposée sur la surface intérieure d'une face du capot 22 et de faces latérales 21, 22. L'antenne 20 est constituée d'une pluralité d'électrodes disposées en matrice, comprenant des électrodes Ei,j situées intégralement sur une face, des électrodes E'i,j disposées en arête sur deux faces, et des électrodes disposées en totalité sur les côtés.

Il est à noter que l'antenne 20 pourrait aussi être disposée sur la surface extérieure du capot du détecteur.

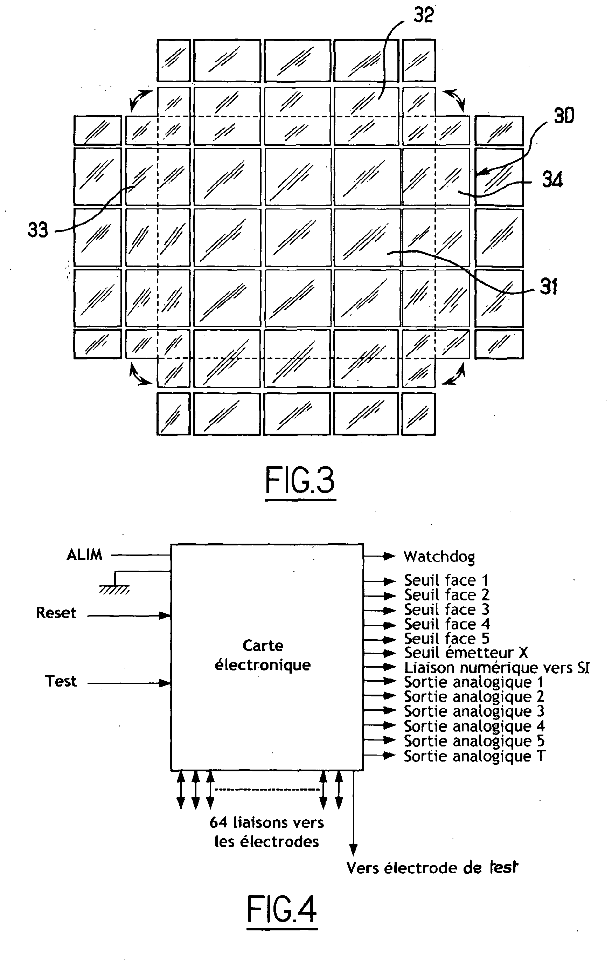

On va maintenant décrire, en référence à la figure 3, un exemple de réalisation d'une antenne 30 sous la forme d'un circuit souple. Cette antenne 30 tapisse la surface intérieure d'une face principale d'un capot avec des électrodes 31 et les surfaces intérieures des faces latérales du capot avec des électrodes 32, 33, 34. Ces électrodes sont toutes reliées à une carte électronique via des pistes conductrices (non représentées).

Les antennes équipant les détecteurs de proximité selon l'invention peuvent être réalisées suivant une technique multicouche, comme l'illustre schématiquement la figure 5. Pour réaliser l'antenne dite X on peut utiliser un circuit imprimé souple 50 composé d'un isolant I métallisé sur ses deux faces d'une couche mince de chrome Cr et d'une couche épaisse de cuivre Cu, les deux couches de cuivre étant supprimées sur une zone ZX qui correspond au passage du faisceau X et dans laquelle des pistes de liaison, des électrodes capacitives Ecr, une piste de test P, et une garde sont réalisées à partir des deux couches de chrome.

Une couche conductrice G de garde en cuivre + chrome, les électrodes Ecu+cr en cuivre + chrome, et les électrodes Ecr en chrome, sont réalisées suivant un processus industriel utilisant des circuits souples multi-couches de type « Adhesiveless », qui possèdent sur un support en polyimide une couche mince de chrome recouverte de cuivre. Ce processus industriel est maîtrisé par les fabricants de circuits souples.

Une carte électronique équipant un détecteur de proximité selon l'invention comprend, en référence à la figure 4, 64 liaisons vers les électrodes de trois antennes de détection, une entrée de test reliée à une électrode de tests pour chaque antenne, une entrée Reset de réinitialisation, et une entrée d'alimentation en tension continue.

Cette carte électronique procure un signal d'alarme «Watchdog», cinq signaux de détection de seuil d'alarme (objets ou patients trop proches), un signal de détection de l'émetteur X, cinq signaux de sortie analogique correspondant aux cinq faces du capot, un signal de sortie analogique de détection de l'émetteur X, un signal d'excitation de l'électrode de test, et une liaison numérique série pour communiquer avec l'unité centrale de l'équipement.

Le signal d'alarme « Watchdog » est placé au niveau bas en cas de mesures incohérentes, d'absence d'électrode ou de défaillance logicielle. Les sorties analogiques sont des images des distances minimales de la face du détecteur, des côtés du détecteur, ou de l'émetteur. Le signal Reset est un signal de réinitialisation du microcontrôleur. La liaison numérique fournit les 64 distances mesurées et l'état de bon fonctionnement du détecteur de proximité. Le capteur est directement branché sur l'unité centrale SI de l'équipement sans carte d'interface.

Comme l'illustre la figure 6, une antenne A d'un détecteur de proximité selon l'invention est reliée via un câble de liaison souple CL à une carte électronique 60 incluant un multiplexeur analogique permettant une scrutation d'entrée, un pont flottant capacitif multivoies mettant en oeuvre une technologie divulguée dans le document FR2756048 correspondant au brevet français n°96 13992 du 15 novembre 1996, un module de conversion analogique/numérique, et un module numérique de calcul des distance, de contrôle de bon fonctionnement et de communication avec le système SI d'information et de commande de la machine.

On va maintenant décrire le fonctionnement d'un détecteur de proximité selon l'invention, en référence aux figures précitées. Le détecteur de proximité mesure une distance successivement sur chaque électrode selon un ordre qui pourra être modifié simplement dans le logiciel.

Le détecteur de proximité dispose d'une électrode de test qui en fonctionnement normal est au potentiel de la garde et qui lorsqu'elle est mise à la masse permet de tester le bon fonctionnement du capteur et le bon état de l'ensemble connectique +antenne.

En agissant sur la commande de test, elle va aussi tester le bon fonctionnement de la chaîne de mesure pour chaque capteur.

Si une distance mesurée sur l'une des électrodes atteint un seuil bas prédéfini, la sortie logique correspondant à l'antenne qui supporte l'électrode passe à l'état bas, elle repassera à l'état haut quand la distance dépassera à nouveau le seuil. La sortie d'alarme « watchdog » passe à l'état bas si l'une des n mesures est incohérente (échec du test) ou si le micro-contrôleur est bloqué ou défaillant.

Dans un premier temps, les sorties numériques sont les images des distances mesurées pour chaque électrodes, mais le traitement peut ensuite devenir plus complexe, il convient donc de prévoir une réserve de puissance de calcul dans le micro-contrôleur (ou DSP).

On va maintenant décrire un exemple pratique de fabrication d'un détecteur de proximité selon l'invention.

Dans cet exemple pratique, le module électrique est disposé sur une carte de longueur 160 mm et de largeur 100 à 160 mm, et comporte un connecteur pour les sorties analogiques (torsadées blindées), un connecteur pour les entrées/sorties logiques, un connecteur pour l'alimentation, et plusieurs connecteurs pour les signaux d'électrodes.

Les antennes qui occupent les arêtes du détecteur auront la moitié de leur surface sur le côté et l'autre sur la grande face. Il y a 33 électrodes réparties sur les 4 antennes : 3 antennes pour le détecteur et une antenne pour l'émetteur X. 13 électrodes sont situées sur les antennes de côté du détecteur, 16 sur l'antenne X et 2 sur l'émetteur X.

La portée des capteurs est supérieure à 100 mm avec une résolution millimétrique, ce qui permet d'optimiser le contrôle de la vitesse d'approche du détecteur vers le patient (vitesse maximale avec risque d'impact minimal).

Les câbles qui relient l'électronique aux antennes du côté de l'émetteur X subissent des mouvements et doivent accepter en pratique un rayon de courbure de 50mm en dynamique.

Pour permettre le remplacement de l'antenne côté émetteur X ou de l'électronique sans enlever le câble, celui-ci est par exemple équipé d'un connecteur côté antenne et d'un connecteur côté électronique. On peut aussi prévoir une bande de garde sur les côtés du détecteur afin de modifier les lignes de champ des électrodes pour modifier les surfaces équivalentes de ces électrodes et leur étendue de mesure et leur portée.

Comme il s'agit d'un dispositif de sécurité, la distance de détection doit être très fiable et le système doit pouvoir être averti en cas de défaillance. Dans les conditions réelles les abords de l'équipement sont très encombrés. Les objets à détecter sont de natures différentes : corps humain (patient couché sur le matelas de la table ou médecin debout à côté de la table), pièces métalliques à la masse ou non, pièces non métalliques mais légèrement conductrices. La détection doit se faire dans n'importe quelle direction. La détection se fait sur toute la surface active du détecteur et sur ses rebords, ce qui correspond à cinq des surfaces d'une boîte.

L'antenne X doit être quasi transparente aux rayons X, ce qui implique pour la réalisation des électrodes et de la garde l'utilisation d'un métal de faible épaisseur. Les médecins installent généralement une protection en plastique léger sur le détecteur (charlotte). L'ordre de grandeur du temps dans lequel une proximité complète doit être détectée est de 50 ms pour une antenne de 64 électrodes. La taille des objets à détecter est variable : de l'abdomen du patient jusqu'à sa main, un doigt ou son nez.

Bien sûr, l'invention n'est pas limitée aux exemples qui viennent d'être décrits et de nombreux aménagements peuvent être apportés à ces exemples sans sortir du cadre de l'invention. Plus généralement, des détecteurs de proximité selon l'invention peuvent être mis en oeuvre dans toute application industrielle, lorsqu'il s'agit de détecter des formes complexes ou une présence à l'aide d'antennes multi-électrodes. On peut ainsi prévoir des détecteurs de proximité selon l'invention équipant des robots mobiles ou des véhicules de transport, pour améliorer la sécurité autour de ces équipements. Des détecteurs de proximité selon l'invention peuvent aussi être mis en oeuvre dans des dispositifs anti-effraction et dans des dispositifs anti-collision.

## Revendications

1. Détecteur de proximité (1A,1B) par capteur capacitif pour piloter l'approche d'une machine (10) et/ou effectuer une détection de sécurité anticollision comprenant:
- au moins une antenne de détection (20,30) comprenant une pluralité de capteurs capacitifs de proximité comportant chacun une électrode de mesure (E_{i,j},31,32,33), ladite au moins une antenne (20,30) étant destinée à être installée dans une pièce mobile (5,2) en déplacement à proximité d'un objet ou d'un corps (3),
- des moyens électroniques pour exciter lesdites électrodes de mesure et pour traiter les signaux issus desdits capteurs capacitifs, et
- des moyens numériques pour piloter les moyens électroniques et pour calculer, à partir des signaux de mesure ainsi traités, des distances entre chacune desdites électrodes de mesure et ledit objet ou ledit corps ;
**caractérisé en ce que**
- lesdites électrodes de mesure (E_{i,j}, 31,32,33) sont disposées en matrice, et
- les moyens électroniques comprennent, pour chaque antenne de détection (20,30), un pont capacitif flottant ou à excitation flottante coopérant avec des moyens de scrutation pour mesurer séquentiellement les capacitances respectives entre chaque électrode (E_{i,j},31,32,33) de ladite antenne (20,30) et l'objet ou le corps (3) à mesurer.

2. Détecteur de proximité (1A,1B) selon la revendication 1, **caractérisé en ce que** l'antenne de détection (20,30) comprend en outre une garde unique (G) pour l'ensemble des électrodes de mesure (E_{i,j},31,32,33) de l'antenne (20,30).

3. Détecteur de proximité (1A,1B) selon la revendication 1, **caractérisé en ce que** l'antenne de détection comprend en outre plusieurs gardes prévues chacune pour une partie de l'ensemble des électrodes de mesure de l'antenne.

4. Détecteur de proximité (1A,1B) selon l'une des revendications précédentes, **caractérisé en ce que** les moyens électroniques et les moyens numériques de pilotage et de calcul coopèrent pour mesurer une distance successivement sur chaque électrode (E_{i,j},31,32,33) d'une antenne (20,30), selon un ordre prédéterminé et modifiable.

5. Détecteur de proximité (1A,1B) selon l'une des revendications précédentes, **caractérisé en ce que** l'une au moins de ses antennes (20,30) de détection comporte une piste de test qui, en fonctionnement normal, est au potentiel de la garde, et, en test, est mise à la masse.

6. Détecteur de proximité (1A,1B) selon la revendication 5, **caractérisé en ce que** la piste de test est placée à l'arrière ou à proximité des électrodes.

7. Détecteur de proximité (1A,1B) selon l'une des revendications précédentes, **caractérisé en ce que** les moyens électroniques et les moyens numériques de pilotage et de calcul coopèrent pour délivrer un signal d'alarme indiquant une mesure incohérente ou un dysfonctionnement des moyens numériques de pilotage et de calcul.

8. Détecteur de proximité (1A,1B) selon l'une des revendications précédentes, **caractérisé en ce que** les moyens électroniques comprennent en outre une ou plusieurs capacités de référence prévues pour contrôler la calibration desdits moyens électroniques ou d'effectuer une recalibration desdits moyens électroniques.

9. Détecteur de proximité (1A,1B) selon l'une des revendications précédentes, **caractérisé en ce qu'**une antenne (20,30) comprend en outre, à proximité des électrodes de mesure, une ou plusieurs surfaces de garde ou de masse qui sont agencées pour modifier les lignes de champ des électrodes de mesure.

10. Détecteur de proximité (1A,1B) selon l'une des revendications précédentes, **caractérisé en ce qu'**il est disposé sur la surface intérieure ou extérieure d'un capot ou boîtier et comprend une pluralité de zones de mesure équipées d'antennes de détection.

11. Détecteur de proximité (1A,1B) selon l'une des revendications précédentes, **caractérisé en ce que** les moyens électroniques et les moyens numériques de pilotage et de calcul coopèrent pour délivrer des signaux de seuil de détection de proximité.

12. Détecteur de proximité (1A,1B) selon l'une des revendications 10 ou 11, **caractérisé en ce que** les moyens électroniques et les moyens numériques de pilotage et de calcul coopèrent pour délivrer des signaux de sortie analogique images de distances minimales entre des zones du boîtier et des objets détectés.

13. Détecteur de proximité (1A,1B) selon l'une des revendications 10 à 12, **caractérisé en ce que** des antennes sont disposées sur cinq faces du boîtier ou capot.

14. Détecteur de proximité (1A,1B) selon l'une des revendications 10 à 13, caractérisé en qu'il comporte des antennes d'arête disposées pour partie sur une face dudit capot, et pour partie sur une autre face (21,22) contiguë, et des antennes latérales.

15. Détecteur de proximité (1A,1B) selon l'une des revendications précédentes, **caractérisé en ce que** l'une au moins des antennes (20,30) est réalisée à l'aide d'un circuit souple.

16. Détecteur de proximité (1A,1B) selon l'une des revendications précédentes, **caractérisé en ce qu'**au moins une des antennes (20,30) est connectée aux moyens électroniques (60) par des moyens de liaison souples (CL).

17. Détecteur de proximité (1A,1B) selon l'une des revendications précédentes, mis en oeuvre dans un équipement de radiologue par rayons X (10) comprenant un dispositif (2) pour émettre un faisceau X prévu pour irradier un objet ou un corps (3) et un dispositif (5) pour détecter le faisceau X issu dudit objet ou corps (3), ce dispositif détecteur X (5) étant recouvert d'un capot, **caractérisé en ce que** le détecteur est disposé sur la surface intérieure ou extérieure dudit capot, dans le champs d'émission X et **en ce qu'**il comporte au moins une antenne, dite antenne X, traversée par le faisceau X.

18. Détecteur de proximité (1A,1B) selon la revendication 17, **caractérisé en ce que** l'antenne X comporte un perçage prévu pour le passage du faisceau X.

19. Détecteur de proximité (1A,1B) selon la revendication 17, **caractérisé en ce que** l'antenne X est réalisée à partir d'un circuit imprimé souple composé d'un isolant métallisé (I) sur ses deux faces d'une couche mince de chrome puis d'une couche de cuivre, ladite couche de cuivre étant supprimée sur une zone qui correspond au passage du faisceau X et dans laquelle des pistes de liaison et des électrodes capacitives sont réalisées à partir de la couche de chrome.

20. Détecteur de proximité (1A,1B) selon l'une des revendications précédentes, mis en oeuvre dans un équipement de radiologue par rayons X (10) comprenant un dispositif (2) pour émettre un faisceau X prévu pour irradier un objet ou un corps (3), **caractérisé en ce qu'**il est disposé sur la surface intérieure ou extérieure dudit dispositif émetteur.

21. Application d'un détecteur de proximité (1A,1B) selon l'une des revendications précédentes, pour le pilotage d'un positionneur vasculaire.

22. Application d'un détecteur de proximité (1A,1B) selon l'une des revendications 1 à 20, dans une machine de radiologie, pour le contrôle de la dose X émise sur un objet ou un corps (3) à partir d'un calcul de l'épaisseur dudit objet ou corps.

23. Application selon la revendication 22, dans laquelle un calcul de l'épaisseur de l'objet ou du corps (3) est effectué à partir de mesures de distances.

24. Application d'un détecteur de proximité selon l'une des revendications 1 à 20, pour le contrôle en vitesse et/ou en position d'une machine en mouvement, notamment une machine-outil.

25. Application d'un détecteur de proximité selon l'une des revendications 1 à 20, pour la détection d'une forme complexe ou d'une présence.

26. Application selon la revendication 25, dans laquelle un ou plusieurs détecteurs de proximité selon l'une des revendications 1 à 20 sont installés dans un équipement ou un véhicule mobile.

27. Application selon la revendication 25, dans un détecteur anti-effraction.

28. Application d'au moins un détecteur de proximité selon l'une des revendications 1 à 20, pour la réalisation d'une caméra capacitive pour la mesure d'une topographie d'objet ou de corps humain.

29. Application d'au moins un détecteur de proximité selon l'une des revendications 1 à 20, pour la réalisation d'image trois dimensions de corps humains.

30. Application d'au moins un détecteur de proximité selon l'une des revendications 1 à 20, pour la détection d'une partie de corps humain telle qu'une main.

## Patentansprüche

1. Näherungssensor (1A, 1B) über einen kapazitiven Sensor zur Ansteuerung der Annäherung einer Maschine (10) und/oder zur Durchführung einer Sicherheitserkennung zum Kollisionsschutz, umfassend:
- mindestens eine Detektionsantenne (20, 30) mit einer Vielzahl von kapazitiven Näherungssensoren mit jeweils einer Messelektrode (E_{i,j}, 31, 32, 33), wobei die mindestens eine Antenne (20, 30) dazu bestimmt ist, in einem beweglichen, in der Nähe eines Gegenstands oder eines Körpers (3) bewegten Element (5, 2) eingebaut zu sein,
- elektronische Einrichtungen zur Erregung der Messelektroden und zur Verarbeitung der Signale aus den kapazitiven Sensoren, und
- digitale Einrichtungen zur Ansteuerung der elektronischen Einrichtungen und anhand der somit verarbeiteten Messsignale zur Berechnung der jeweiligen Abstände zwischen den einzelnen Messelektroden und dem Gegenstand oder dem Körper;
**dadurch gekennzeichnet, dass**:
- die Messelektroden (E_{i,j}, 31, 32, 33) als Matrix angeordnet sind, und
- die elektronischen Einrichtungen für jede Detektionsantenne (20, 30) eine potentialfreie, kapazitive Brücke oder mit potentialfreier Erregung umfassen, die mit Abtastungsmitteln zur sequentiellen Messung der jeweiligen Kapazitanzen zwischen jeder Elektrode (E_{i,j}, 31, 32, 33) der Antenne (20, 30) und dem zu messenden Gegenstand oder Körper (3) zusammenwirkt.

2. Näherungssensor (1A, 1B) nach Anspruch 1, **dadurch gekennzeichnet, dass** die Detektionsantenne (20, 30) außerdem eine einzige Schirmelektrode (G) für die gesamten Messelektroden (E_{i,j}, 31, 32, 33) der Antenne (20, 30) umfasst.

3. Näherungssensor (1A, 1B) nach Anspruch 1, **dadurch gekennzeichnet, dass** die Detektionsantenne außerdem mehrere Schirmelektroden umfasst, die jeweils für einen Teil der gesamten Messelektroden der Antenne vorgesehen sind.

4. Näherungssensor (1A, 1B) nach einem der vorhergehenden Ansprüche, **dadurch gekennzeichnet, dass** die elektronischen Einrichtungen und die digitalen Ansteuerungs- und Berechnungseinrichtungen zusammenwirken, um in einer vorbestimmten und änderbaren Reihenfolge einen Abstand an jeder Elektrode (E_{i,j}, 31, 32, 33) einer Antenne (20, 30) nacheinander zu messen.

5. Näherungssensor (1A, 1B) nach einem der vorhergehenden Ansprüche, **dadurch gekennzeichnet, dass** mindestens eine dieser Detektionsantennen (20, 30) einen Teststreifen beinhaltet, der im normalen Betrieb auf das Potenzial der Schirmelektrode gelegt und im Testbetrieb geerdet wird.

6. Näherungssensor (1A, 1B) nach Anspruch 5, **dadurch gekennzeichnet, dass** der Teststreifen an der Rückseite oder in der Nähe der Elektroden gelegt wird.

7. Näherungssensor (1A, 1B) nach einem der vorhergehenden Ansprüche, **dadurch gekennzeichnet, dass** die elektronischen Einrichtungen und die digitalen Ansteuerungs- und Berechnungseinrichtungen zusammenwirken, um bei einem inkohärenten Messwert oder einer Funktionsstörung der digitalen Ansteuerungs- und Berechnungseinrichtungen einen Warnsignal auszugeben.

8. Näherungssensor (1A, 1B) nach einem der vorhergehenden Ansprüche, **dadurch gekennzeichnet, dass** die elektronischen Einrichtungen außerdem eine oder mehrere Referenzkapazitäten zur Überprüfung der Kalibrierung der elektronischen Einrichtungen oder zur Durchführung einer Neukalibrierung der elektronischen Einrichtungen umfassen.

9. Näherungssensor (1A, 1B) nach einem der vorhergehenden Ansprüche, **dadurch gekennzeichnet, dass** eine Antenne (20, 30) außerdem in der Nähe der Messelektroden eine oder mehrere Schirmflächen oder geerdete Oberflächen umfasst, welche derart angeordnet sind, um die Feldlinien der Messelektroden zu ändern.

10. Näherungssensor (1A, 1B) nach einem der vorhergehenden Ansprüche, **dadurch gekennzeichnet, dass** er an der inneren oder äußeren Oberfläche einer Schutzhaube oder eines Gehäuses angeordnet ist und eine Vielzahl an mit Detektionsantennen ausgestatteten Messbereichen umfasst.

11. Näherungssensor (1A, 1B) nach einem der vorhergehenden Ansprüche, **dadurch gekennzeichnet, dass** die elektronischen Einrichtungen und die digitalen Ansteuerungs- und Berechnungseinrichtungen zusammenwirken, um Signale bei Erkennung einer Näherungsgrenze auszugeben.

12. Näherungssensor (1A, 1B) nach einem der Ansprüche 10 oder 11, **dadurch gekennzeichnet, dass** die elektronischen Einrichtungen und die digitalen Ansteuerungs- und Berechnungseinrichtungen zusammenwirken, um analoge Ausgangssignale als Darstellungen von Mindestabständen zwischen Bereichen des Gehäuses und erfassten Gegenständen auszugeben.

13. Näherungssensor (1A, 1B) nach einem der Ansprüche 10 bis 12, **dadurch gekennzeichnet, dass** Antennen an fünf Seiten des Gehäuses oder der Schutzhaube angeordnet sind.

14. Näherungssensor (1A, 1B) nach einem der Ansprüche 10 bis 13, **dadurch gekennzeichnet, dass** er kantenseitige Antennen, die zum Teil an einer Seite der Schutzhaube und zum Teil an einer anderen, angrenzenden Seite (21, 22) angeordnet sind, sowie seitliche Antennen beinhaltet.

15. Näherungssensor (1A, 1B) nach einem der vorhergehenden Ansprüche, **dadurch gekennzeichnet, dass** mindestens eine der Antennen (20, 30) anhand einer biegsamen Schaltung ausgeführt ist.

16. Näherungssensor (1A, 1B) nach einem der vorhergehenden Ansprüche, **dadurch gekennzeichnet, dass** mindestens eine der Antennen (20, 30) mit den elektronischen Einrichtungen (60) über biegsame Verbindungseinrichtungen (CL) verbunden ist.

17. Näherungssensor (1A, 1B) nach einem der vorhergehenden Ansprüche, in einem Radiologiegerät über Röntgenstrahlung (10) eingesetzt, welches eine Vorrichtung (2) zum Senden einer Röntgenstrahlung zur Bestrahlung eines Gegenstands oder eines Körpers (3) sowie eine Vorrichtung (5) zur Erkennung der Röntgenstrahlung aus dem Gegenstand oder Körper (3) umfasst, wobei die Vorrichtung zur Röntgenerkennung (5) mit einer Schutzhaube bedeckt ist, **dadurch gekennzeichnet, dass** der Sensor an der inneren oder äußeren Oberfläche der Schutzhaube im Röntgen-Emissionsfeld angeordnet ist und dadurch, dass er mindestens eine sogenannte Röntgenantenne umfasst, durch welche die Röntgenstrahlung hindurch strahlt.

18. Näherungssensor (1A, 1B) nach Anspruch 17, **dadurch gekennzeichnet, dass** die Röntgenantenne eine Bohrung für den Durchgang der Röntgenstrahlung umfasst.

19. Näherungssensor (1A, 1B) nach Anspruch 17, **dadurch gekennzeichnet, dass** die Röntgenantenne anhand einer biegsamen gedruckten Schaltung ausgeführt ist, welche aus einem auf seinen beiden Seiten mit einer dünnen Chromschicht und anschließend mit einer Kupferschicht metallisierten Isolator (I) besteht, wobei die Kupferschicht an einem Bereich entfernt ist, der dem Durchgang der Röntgenstrahlung entspricht und an welchem Verbindungsstreifen und kapazitive Elektroden anhand der Chrom-Schicht ausgeführt sind.

20. Näherungssensor (1A, 1B) nach einem der vorhergehenden Ansprüche, in einem Radiologiegerät mit Röntgenstrahlung (10) eingesetzt, das eine Vorrichtung (2) zum Senden einer Röntgenstrahlung zur Bestrahlung eines Gegenstands oder eines Körpers (3) umfasst, **dadurch gekennzeichnet, dass** er an der inneren oder äußeren Oberfläche der Sendevorrichtung angeordnet ist.

21. Anwendung eines Näherungssensors (1A, 1B) nach einem der vorhergehenden Ansprüche zur Ansteuerung einer vaskulären Positioniereinrichtung.

22. Anwendung eines Näherungssensors (1A, 1B) nach einem der Ansprüche 1 bis 20 in einer Radiologiemaschine zur Überprüfung der Röntgenmenge, die auf einen Gegenstand oder einen Körper (3) anhand einer Berechnung der Dicke des Gegenstands oder Körpers gestrahlt wird.

23. Anwendung nach Anspruch 22, wobei eine Berechnung der Dicke des Gegenstands oder des Körpers (3) anhand von Abstandsmessungen erfolgt.

24. Anwendung eines Näherungssensors nach einem der Ansprüche 1 bis 20 zur Überprüfung der Geschwindigkeit und/oder der Position einer bewegten Maschine, insbesondere einer Werkzeugmaschine.

25. Anwendung eines Näherungssensors nach einem der Ansprüche 1 bis 20 zur Erfassung einer komplexen Form oder einer Präsenz.

26. Anwendung nach Anspruch 25, wobei ein oder mehrere Näherungssensoren nach einem der Ansprüche 1 bis 20 in einer Einrichtung oder einem fahrbaren Fahrzeug eingebaut sind.

27. Anwendung nach Anspruch 25 in einem Einbruchsmelder.

28. Anwendung mindestens eines Näherungssensors nach einem der Ansprüche 1 bis 20 für die Ausführung einer kapazitiven Kamera zur Messung einer Topografie eines Gegenstands oder eines menschlichen Körpers.

29. Anwendung mindestens eines Näherungssensors nach einem der Ansprüche 1 bis 20 für die Ausführung von 3D-Bildern menschlicher Körper.

30. Anwendung mindestens eines Näherungssensors nach einem der Ansprüche 1 bis 20 zur Erfassung eines menschlichen Körperteils wie einer Hand.

## Claims

1. Proximity detector (1A, 1B) employing a capacitive sensor for controlling the approach of a machine (10) and/or performing a safety anti-collision detection, comprising:
- at least one detection antenna (20, 30) comprising a plurality of capacitive proximity sensors, each comprising a measurement electrode (E_{i,j}, 31, 32, 33), said at least one antenna (20, 30) being intended to be placed in a moving part (5, 2) moving close to an object or a body (3),
- electronic means for exciting said measurement electrodes and for processing the signals originating from said capacitive sensors,
- digital means for controlling the electronic means and for calculating, from the measurement signals thus processed, the distances between said electrodes and said object or said body,
**characterised in that**
- said measurement electrodes (E_{i,j}, 31, 32, 33) are arranged as a matrix, and
- the electronic means comprise, for each detection antenna (20, 30), a floating capacitive bridge or with floating excitation, cooperating with polling means to measure sequentially the respective capacitances between each electrode (E_{i,j}, 31, 32, 33) of said antenna (20, 30) and the object or body to be measured.

2. Proximity detector (1A, 1B) according to claim 1, **characterised in that** the detection antenna (20, 30) also comprises a single shield (G) for all the measurement electrodes (E_{i,j}, 31, 32, 33) of the antenna (20, 30).

3. Proximity detector (1A, 1B) according to claim 1, **characterised in that** the detection antenna also comprises a number of shields each provided for a part of the assembly of measurement electrodes of the antenna.

4. Proximity detector (1A, 1B) according to one of the preceding claims, **characterised in that** the electronic means and the digital control and calculation means cooperate to measure a distance successively on each electrode (E_{i,j}, 31, 32, 33) of an antenna (20, 30) according to a predetermined but changeable order.

5. Proximity detector (1A, 1B) according to one of the preceding claims, **characterised in that** at least one of its detection antennas (20, 30) comprises a test track which, in normal operation, is at the potential of the shield and, in test mode, is earthed.

6. Proximity detector (1A, 1B) according to claim 5, **characterised in that** the test track is placed to the rear of or close to the electrodes.

7. Proximity detector (1A, 1B) according to one of the preceding claims, **characterised in that** the electronic means and the digital control and calculation means cooperate to deliver an alarm signal indicating an inconsistent measurement or a malfunction of the digital control and calculation means.

8. Proximity detector (1A, 1B) according to one of the preceding claims, **characterised in that** the electronic means also comprise one or more reference capacitances provided to check the calibration of said electronic means or to recalibrate said electronic means.

9. Proximity detector (1A, 1B) according to one of the preceding claims, **characterised in that** one antenna (20, 30) also comprises, close to the measurement electrodes, one or more shield or earthing surfaces which are arranged to modify the field lines of the measurement electrodes.

10. Proximity detector (1A, 1B) according to one of the preceding claims, **characterised in that** it is arranged on the inside or outside surface of a cap or box and comprises a plurality of measurement areas equipped with detection antennas.

11. Proximity detector (1A, 1B) according to one of the preceding claims, **characterised in that** the electronic means and the digital control and calculation means cooperate to deliver proximity detection threshold signals.

12. Proximity detector (1A, 1B) according to one of claims 10 and 11, **characterised in that** the electronic means and the digital control and calculation means cooperate to deliver analogue output signals of minimum distance images between the zones of the box and the objects detected.

13. Proximity detector (1A, 1B) according to one of claims 10 to 12, **characterised in that** the antennas are arranged on five faces of the box or cap.

14. Proximity detector (1A, 1B) according to one of claims 10 to 13, **characterised in that** it comprises edge antennas arranged in part over one face of said cap, and in part over another contiguous face (21, 22), and lateral antennas.

15. Proximity detector (1A, 1B) according to one of the preceding claims, **characterised in that** at least one of the antennas (20, 30) is produced using a flexible circuit.

16. Proximity detector (1A, 1B) according to one of the preceding claims, **characterised in that** at least one of the antennas (20, 30) is connected to the electronic means (60) by flexible connecting means (CL).

17. Proximity detector (1A, 1B) according to one of the preceding claims, used in radiology equipment employing X-rays (10), comprising a device (2) for emitting an X-ray beam provided to irradiate an object or a body (3) and a device (5) for detecting the X-rays originating from said object or body (3), this X-ray detector device (5) being covered by a cap, **characterised in that** the detector is arranged on the inside or outside surface of said cap, in the X-ray emission field and **in that** it comprises at least one antenna, termed the X-ray antenna, crossed by the X-ray beam.

18. Proximity detector (1A, 1B) according to claim 17, **characterised in that** the X-ray antenna comprises a piercing provided for the passage of the X-ray beam.

19. Proximity detector (1A, 1B) according to claim 17, **characterised in that** the X-ray antenna is produced from a flexible printed circuit composed of an insulator (I) metallised on both of its faces with a thin layer of chromium then by a layer of copper, said copper layer being removed over an area which corresponds to the passage of the X-ray beam and in which the linking tracks and the capacitive electrodes are produced from the chromium layer.

20. Proximity detector (1A, 1B) according to one of the preceding claims, fitted in radiology equipment employing X-rays (10), comprising a device (2) for emitting an X-ray beam intended to irradiate an object or a body (3), **characterised in that** it is arranged on the inside or outside surface of said emitter device.

21. Application of a proximity detector (1A, 1B) according to one of the preceding claims, for controlling a vascular positioner.

22. Application of a proximity detector (1A, 1B) according to one of claims 1 to 20, in a radiology machine, for checking the X-ray dose emitted on to an object or a body (3), starting from a calculation of the thickness of said object or body.

23. Application according to claim 22, in which the thickness of the object or body (3) is calculated from distance measurements.

24. Application of a proximity detector according to one of claims 1 to 20, for checking the speed and/or position of a machine in motion, in particular a machine tool.

25. Application of a proximity detector according to one of claims 1 to 20, for detecting a complex shape or a presence.

26. Application according to claim 25, in which one or more proximity detectors according to one of claims 1 to 20 are installed in an item of equipment or a moving vehicle.

27. Application according to claim 25, in an anti-burglary detector.

28. Application of at least one proximity detector according to one of claims 1 to 20, for the production of a capacitive camera for the measurement of a object or human body topography.

29. Application of at least one proximity detector according to one of claims 1 to 20, for the production of image in three dimensions of human body.

30. Application of at least one proximity detector according to one of claims 1 to 20, for the detection of a part of human body as a hand.
